# EUROPEAN PATENT SPECIFICATION

(11) **EP 2 520 294 B1**
(45) Date of publication and mention of the grant of the patent: **18.02.2015**
(21) Application number: 10841215.6
(22) Date of filing: 27.12.2010
(51) Int. Cl.: A61K 31/352, A61P 27/16, A61K 31/343, A61K 31/39

(54) **COMPOSITION INCLUDING A NAPHTHOQUINONE DERIVATIVE FOR TREATING AND PREVENTING HEARING LOSS**
ZUSAMMENSETZUNG MIT EINEM NAPHTHOCHINONDERIVAT ZUR BEHANDLUNG UND VERHINDERUNG VON HÖRVERLUST
COMPOSITION COMPRENANT UN DERIVE DE LA NAPHTOQUINONE POUR TRAITER ET PRÉVENIR LA PERTE D'AUDITION

(30) Priority: 28.12.2009 KR 20090131810
(43) Date of publication of application: 07.11.2012
(73) Proprietor: KT&G Life Sciences Corporation, Daejeon 306-712 (KR)
(72) Inventor: KWAK, Tae Hwan, Yongin-si Gyeonggi-do 446-906 (KR); PARK, Myung-Gyu, Yongin-si Gyeonggi-do 446-908 (KR); SO, Hong-Seob, Iksan-si Jeonbuk 570-763 (KR); PARK, Raekil, Iksan-si Jeonbuk 570-160 (KR); KIM, Hyung-Jin, Iksan-si Jeonbuk 570-160 (KR)
(74) Representative: Jansen, Cornelis Marinus
(86) International application number: PCT/KR2010/009369
(87) International publication number: WO 2011/081383

(56) References cited:
- WO-A1-2008/066295
- WO-A1-2008/066300
- WO-A2-2009/140382
- US-A1- 2004 204 471

## Description

### [Technical Field]

The present invention relates to a composition for treating or preventing hearing loss comprising naphthoquinone-based compounds.

### [Background Art]

The Corti's organ, which is an auditory epithelial group on basilar membrane in the cochlear (cochlear duct) of the inner ear, functions through auditory hair cells which are the lowest number of sensory receptor cells among all sensory tissues. A human cochlea has only 20,000 sensory receptor cells, as compared to retina having 137,000,000 visual sensory receptor cells. Mammalian auditory hair cells are produced only during embryogenesis, which are not reproduced in the case of being lost after birth. Accordingly, the loss of the hair cells causes obvious and irreversible hearing loss.

It has been found that the hearing loss is mainly caused by the death of the auditory hair cells, which is induced from various causes including trauma, aging, excessive noise and pollution, ototoxic drugs such as antibiotics (e.g., gentamicin), loop diuretics and platinum-based chemotherapy agents (e.g., cisplatin), infection, autoimmune diseases and hereditary diseases.

Recently, there have been several studies for developing an agent effective in preventing and treating the hearing loss. As a result, antioxidants, N-methyl-D-aspartate (NMDA) antagonists, apoptosis inhibitors, etc., have been reported, but there is still no approved drug for preventing and treating the hearing loss.

Meanwhile, naphthoquinone-based compounds are known as active ingredients of some pharmaceutical compositions. Among them, β-lapachone is obtained from the laphacho tree (*Tabebuia avellanedae*) in South America, and dunnione and α-dunnione are obtained from the leaves of *Streptocarpus dunnii.* These natural tricyclic naphthoquinone derivatives are well used as a therapeutic agent against Chagas disease which is endemic in South America, as well as an anti-cancer agent for a long time, which are also known to exhibit superior effects. Particularly, as the pharmacological effects as an anti-cancer agent are known to the West, the compounds attracted much attention. As disclosed in US Patent No. 5,969,163, the tricyclic naphthoquinone derivatives have been developed as various anti-cancer agents.

WO 2008/066300 describes naphthoquinone-based compounds that can be used for treatment or prevention of obesity, diabetes, metabolic syndrome, neurodegenerative diseases and mitochondrial dysfunction diseases.

WO 2008/066295 describes pharmaceutical compositions comprising naphthoquinone-based compounds that can be used for treatment or prevention of metabolic diseases, degenerative diseases and mitochondrial dysfunction-related diseases.

US 2004/0204471 describes a method of preventing or treating otic disorders and otic disorder-related complications in a subject comprising administering to the subject a Cox-2 inhibitor.

### [Disclosure]

### [Technical Problem]

The present inventors have endeavored to develop a drug for treating or preventing hearing loss due to aging, ototoxic drugs, etc., and found that naphthoquinone-based compounds are effective in the treatment and prevention of hearing loss.

An object of the present invention is, therefore, to provide a composition for treating or preventing hearing loss selected from age-related presbycusis and ototoxic drug-related hearing loss comprising naphthoquinone-based compounds.

### [Technical Solution]

The present invention relates to a composition comprising a compound of Formula 1 or 2, or a pharmaceutically acceptable salt, solvate or isomer thereof for use in treating or preventing hearing loss selected from age-related presbycusis and ototoxic drug-related hearing loss: 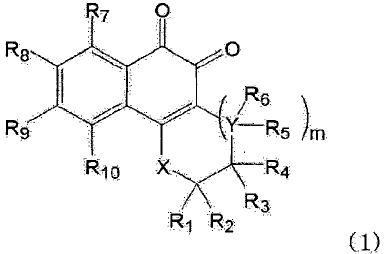 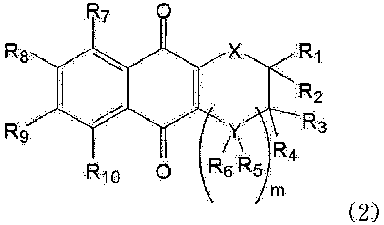 wherein,
R₁ to R₆ are each independently selected from the group consisting of hydrogen, hydroxy, halogen, amino, substituted or unsubstituted C₁-C₁₀ alkylamino, substituted or unsubstituted C₁-C₁₀ dialkylamino, substituted or unsubstituted C₁-C₁₀ alkyl, substituted or unsubstituted C₂-C₁₀ alkenyl, substituted or unsubstituted C₂-C₁₀ alkynyl, substituted or unsubstituted C₁-C₁₀ alkoxy, substituted or unsubstituted C₁-C₁₀ alkoxycarbonyl, substituted or unsubstituted C₁-C₁₀ acyl, substituted or unsubstituted C₃-C₈ cycloalkyl, substituted or unsubstituted C₃-C₈ heterocycloalkyl, substituted or unsubstituted C₄-C₁₀ aryl, substituted or unsubstituted C₄-C₁₀ heteroaryl, and substituted or unsubstituted -(CH₂)ₙ-aryl, or two substituents of R₁ to R₆ may be taken together to form a double bond or a substituted or unsubstituted C₃-C₆ cyclic structure which may be saturated or partially or completely unsaturated; the substituent being at least one selected from the group consisting of hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₁-C₁₀ alkylamino, C₃-C₈ cycloalkyl, C₃-C₈ heterocycloalkyl, C₄-C₁₀ aryl, and C₄-C₁₀ heteroaryl;
R₇ to R₁₀ are each independently selected from the group consisting of hydrogen, hydroxy, halogen, amino, substituted or unsubstituted C₁-C₁₀ alkylamino, substituted or unsubstituted C₁-C₁₀ dialkylamino, substituted or unsubstituted C₁-C₁₀ alkyl, substituted or unsubstituted C₂-C₁₀ alkenyl, substituted or unsubstituted C₂-C₁₀ alkynyl, substituted or unsubstituted C₁-C₁₀ alkoxy, substituted or unsubstituted C₁-C₁₀ alkoxycarbonyl, substituted or unsubstituted C₁-C₁₀ acyl, substituted or unsubstituted C₃-C₈ cycloalkyl, substituted or unsubstituted C₃-C₈ heterocycloalkyl, substituted or unsubstituted C₄-C₁₀ aryl, substituted or unsubstituted C₄-C₁₀ heteroaryl, and substituted or unsubstituted -(CH₂)ₙ-aryl, or two substituents of R₇ to R₁₀ may be taken together to form a substituted or unsubstituted C₃-C₆ cyclic structure which may be saturated or partially or completely unsaturated; the substituent being at least one selected from the group consisting of hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₁-C₁₀ alkylamino, C₃-C₈ cycloalkyl, C₃-C₈ heterocycloalkyl, C₄-C₁₀ aryl, and C₄-C₁₀ heteroaryl;
X is O, S or NR', with R' being hydrogen or C₁-C₆ alkyl;
Y is C, S, N or O, with the proviso that when Y is S or O, R₅ and R₆ are not any substituent, and when Y is N, R₅ is hydrogen or C₁-C₆ alkyl and R₆ is not any substituent;
m is 0 or 1, with the proviso that when m is 0, carbon atoms adjacent to m form a cyclic structure via a direct bond; and
n is an integer of 0 to 10.

Preferably, X is O or S, and Y is C or O.

In a preferred embodiment, R₁ to R₆ are each independently selected from the group consisting of hydrogen, hydroxy, halogen, substituted or unsubstituted C₁-C₁₀ alkyl, substituted or unsubstituted C₂-C₁₀ alkenyl, substituted or unsubstituted C₁-C₁₀ alkoxy, and -(CH₂)ₙ-phenyl, or R₁ and R₄ or R₂ and R₃ may be taken together to form a double bond or a C₄-C₆ cyclic structure, the substituent being C₁-C₁₀ alkyl.

In another preferred embodiment, R₇ to R₁₀ are each independently selected from the group consisting of hydrogen, hydroxy, halogen, substituted or unsubstituted C₁-C₁₀ alkyl, and substituted or unsubstituted C₁-C₁₀ alkoxy.

A preferred embodiment of the compound of Formula 1 or 2 is a compound of Formula 1-1 or 2-1 wherein X is O and Y is C, or a compound of Formula 1-2 or 2-2 wherein X is S: 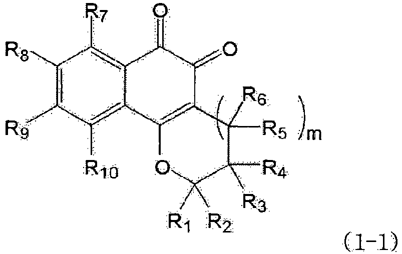 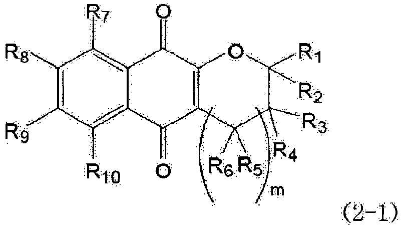 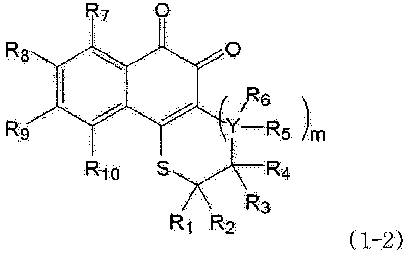 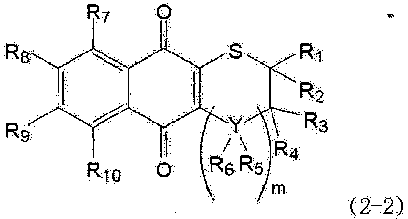 wherein, R₁ to R₁₀, Y and m have the same meanings as defined in Formula 1.

In another preferred embodiment, the compound of Formula 1 or 2 is a compound of Formula 1-3 or 2-3 wherein m is 0 and carbon atoms adjacent to m form a cyclic structure (furan ring) via a direct bond, which is often referred to as "furan compound" or "furano-o-naphthoquinone derivative" hereinafter: 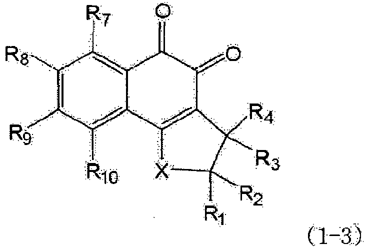 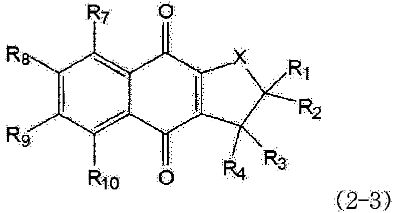 wherein, R₁ to R₁₀, and X have the same meanings as defined in Formula 1.

In a further preferred embodiment, the compound of Formula 1 or 2 is a compound of Formula 1-4 or 2-4 wherein m is 1, which is often referred to as "pyran compound" or " pyrano-o-naphthoquinone derivative" hereinafter: 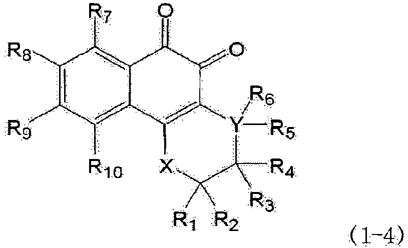 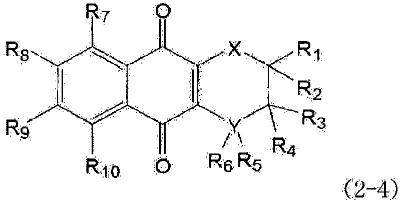 wherein, R₁ to R₁₀, X and Y have the same meanings as defined in Formula 1.

In a further preferred embodiment, the compound of Formula 1 or 2 is a compound of Formula 1-5 or 1-6 or Formula 2-5 or 2-6 wherein R₇ and R₈ are taken together to form a cyclic structure: 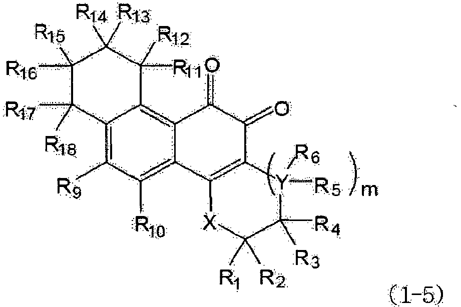 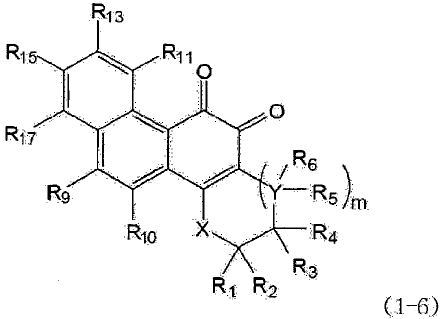 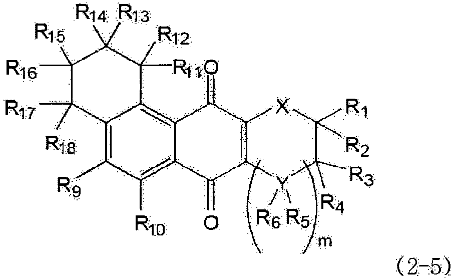 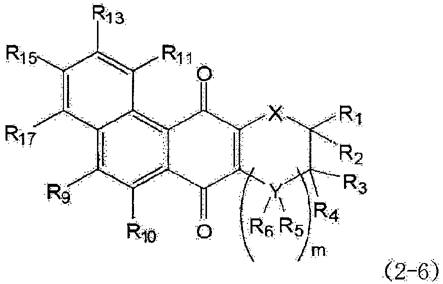 wherein,
R₁ to R₁₀, X, Y and m have the same meanings as defined in Formula 1, and
R₁₁ to R₁₈ are each independently selected from the group consisting of hydrogen, hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₁-C₁₀ alkylamino, C₃-C₈ cycloalkyl, C₃-C₈ heterocycloalkyl, C₄-C₁₀ aryl, and C₄-C₁₀ heteroaryl.

Preferably, R₁₁ to R₁₈ are each independently selected from the group consisting of hydrogen, hydroxy, halogen, C₁-C₁₀ alkyl, C₂-C₁₀ alkenyl, C₂-C₁₀ alkynyl, C₁-C₁₀ alkoxy, C₃-C₈ cycloalkyl, and phenyl.

As used herein, the term "pharmaceutically acceptable salt" means a formulation of a compound that does not cause significant irritation to an organism to which it is administered and does not abrogate the biological activity and properties of the compound. Examples of the pharmaceutically acceptable salt may include addition salts with acids capable of forming non-toxic acid addition salts containing pharmaceutically acceptable anions, for example, inorganic acids such as hydrochloric acid, sulfuric acid, nitric acid, phosphoric acid, hydrobromic acid and hydroiodic acid; organic carbonic acids such as tartaric acid, formic acid, citric acid, acetic acid, trichloroacetic acid, trifluoroacetic acid, gluconic acid, benzoic acid, lactic acid, fumaric acid, maleic acid and salicylic acid; and sulfonic acids such as methanesulfonic acid, ethanesulfonic acid, benzenesulfonic acid and p-toluenesulfonic acid. Meanwhile, base addition salts may include salts with alkali metals or alkaline earth metals such as lithium, sodium, potassium, calcium and magnesium, salts with amino acids such as arginine, lysine and guanidine, and salts with organic bases such as dicyclohexylamine, N-methyl-D-glucamine, tris(hydroxymethyl)methylamine, diethanolamine, choline and triethylamine. The compound of Formula 1 or 2 in accordance with the present invention may be converted into salts thereof by conventional methods well-known in the art.

As used herein, the term "solvate" means a compound of the present invention or a salt thereof, which further includes a stoichiometric or non-stoichiometric amount of a solvent bound thereto by non-covalent intermolecular forces. Preferred solvents are volatile, non-toxic, and/or acceptable for administration to humans. In the case that the solvent is water, the solvate refers to a hydrate.

As used herein, the term "isomer" means a compound of the present invention or a salt thereof, which has the same chemical formula or molecular formula but is optically or stereochemically different therefrom.

Unless otherwise specified, the term "compound of Formula 1 or 2" or "naphthoquinone-based compound" is intended to encompass the compound per se, and the pharmaceutically acceptable salt, solvate and isomer thereof.

As used herein, the term "alkyl" refers to a radical containing carbon and hydrogen without any unsaturated moieties. The alkyl radical may be a straight (linear) or branched chain. Examples of "alkyl" include, but are not limited to, methyl, ethyl, propyl, isopropyl, butyl, isobutyl, t-butyl and sec-butyl.

C₁-C₁₀ alkyl is an alkyl group having 1 to 10 carbon atoms in the straight or branched main chain. The alkyl group may be optionally substituted with 4 or less of substituents at an optional bonding point(s) (optional carbon atom(s)).

Meanwhile, an alkyl group substituted with an alkyl group means a "branched alkyl group".

As used herein, the term "alkenyl" refers to an unsaturated aliphatic group containing at least one carbon-carbon double bond, which is similar to the alkyl group in terms of length and substitutability. Examples of "alkenyl" include a straight alkenyl group (e.g., ethenyl, propenyl, butenyl, pentenyl, hexenyl, heptenyl, octenyl, nonenyl, decenyl), a branched alkenyl group, and cycloalkenyl group (e.g., cyclopropenyl, cyclopentenyl, cyclohexenyl, cycloheptenyl, cyclooctenyl). Also, the "alkenyl" may further include an alkenyl group containing oxygen, nitrogen, sulfur or phosphorus replacing at least one carbon of the hydrocarbon main chain. In a specific example, the straight or branched alkenyl group may have no more than 6 carbon atoms in the main chain (e.g., straight C₂-C₆, branched C₃-C₆). Similarly, the cycloalkenyl group may have 3 to 8 carbon atoms, preferably 5 to 6 carbon atoms, in the ring structure.

As used herein, the term "alkynyl" refers to an unsaturated aliphatic group containing at least one carbon-carbon triple bond, which is similar to the alkyl group in terms of length and substitutability. Examples of "alkynyl" include a straight alkynyl group (e.g., ethynyl, propynyl, butynyl, pentynyl, hexynyl, heptynyl, octynyl, nonynyl, decynyl), and a branched alkynyl group (including an alkynyl group substituted with akyl or alkenyl). Also, the "alkynyl" may further include an alkynyl group containing oxygen, nitrogen, sulfur or phosphorus replacing at least one carbon of the hydrocarbon main chain. In a specific example, the straight or branched alkynyl group may have no more than 6 carbon atoms in the main chain (e.g., straight C₂-C₆, branched C₃-C₆).

The alkyl, alkynyl and alkenyl may be optionally substituted with a substituent such as hydroxy, carboxylate, oxo, halogen (e.g., F, Cl, Br, I), C₁-C₆ haloalkyl (e.g., CCl₃ or CF₃), carbamoyl (-NHCOOR or -OCONHR), urea (-NHCONHR), thiol, cyano, nitro, amino, acylamino, C₁-C₁₀ alkylthio, C₄-C₁₀ arylthio, C₁-C₁₀ alkyl, C₁-C₁₀ alkoxy, C₄-C₁₀ aryloxy, C₁-C₁₀ alkylcarbonyloxy, C₄-C₁₀ arylcarbonyloxy, C₃-C₈ cycloalkyl, C₃-C₈ cycloalkyloxy, C₂-C₁₀ alkenyl, C₂-C₁₀ alkynyl, C₄-C₁₀ aryl, aminocarbonyl, C₁-C₁₀ alkylcarbonyl, C₃-C₈ cycloalkylcarbonyl, C₃-C₈ heterocycloalkylcarbonyl, C₄-C₁₀ arylcarbonyl, C₄-C₁₀ aryloxycarbonyl, C₁-C₁₀ alkoxycarbonyl, C₃-C₈ cycloalkyloxycarbonyl, C₃-C₈ heterocycloalkyloxycarbonyl, C₁-C₁₀ alkylsulfonyl, C₄-C₁₀ arylsulfonyl, C₁-C₁₀ alkylamino, C₃-C₈ heterocycloalkyl and C₄-C₁₀ heteroaryl.

Preferably, the substituent may be at least one selected from hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₁-C₁₀ alkylamino, C₃-C₈ cycloalkyl, C₃-C₈ heterocycloalkyl, C₄-C₁₀ aryl and C₄-C₁₀ heteroaryl.

As used herein, the term "cycolalkyl" means an alkyl group containing 3 to 15 carbon atoms, preferably 3 to 8 carbon atoms without any alternating or resonance double bond between the carbon atoms. The cycloalkyl may include 1 to 4 rings. Examples of "cycloalkyl" include cycloproyl, cyclobutyl, cyclopentyl, cyclohexyl and adamantyl. The cycloalkyl may be substituted with a substituent such as halogen, C₁-C₁₀ alkyl, C₁-C₁₀ alkoxy, amino, nitro, cyano, thiol and C₁-C₁₀ alkylthio.

As used herein, the term "heterocycloalkyl" means a saturated or unsaturated bicyclic seven to eleven-membered heterocyclic ring or a stable monocyclic nonaromatic three to eight-membered heterocyclic ring in which one or more ring carbon atoms are replaced with heteroatoms such as oxygen, nitrogen and sulfur, which may form additional fused-, spiro- or crosslinked-ring. Each heterocyclic ring is consisted of one or more carbon atoms and 1 to 4 hetero atoms. The heterocycloalkyl may be bonded to an optional endocyclic ring which provides a stable structure. Examples of "heterocycloalkyl" may include, but are not limited to, furan, thiophene, pyrrole, pyrroline, pyrrolidine, oxazole, thiazole, imidazole, imidazoline, imidazolidine, pyrazole, pyrazoline, pyrazolidine, isothiazole, triazole, thiadiazole, pyran, pyridine, piperidine, morpholine, thiomorpholine, pyridazine, pyrimidine, pyrazine, piperazine and triazine.

As used herein, the term "aryl" refers to an aromatic group which has at least one ring having a conjugated pi (π) electron system and includes both carbocyclic aryl (for example, phenyl) and heterocyclic aryl (for example, pyridine). This term includes monocyclic or fused-ring polycyclic (i.e., rings which share adjacent pairs of carbon atoms) groups. The aryl may be a carbocyclic group or contain 1 to 4 optional heteroatoms (for example, nitrogen, sulfur or oxygen), which is called "heteroaryl."

Examples of the aryl or heteroaryl may include, but are not limited to, phenyl, naphthyl, pyridyl, pyrimidyl, pyrrolyl, isothiazolyl, triazolyl, tetrazolyl, pyrazolyl, oxazolyl, isooxazolyl, pyrazinyl, pyridazinyl, triazinyl, quinazolynyl, thiazolyl, benzothiophenyl, furanyl, imidazolyl and thiophenyl.

The cycloalkyl, heterocycloalkyl, aryl and heteroaryl may be optionally substituted. Examples of the substitutent may include, but are not limited to, hydroxy, halogen, thiol, cyano, nitro, amino, acylamino, C₁-C₁₀ alkylthio, C₄-C₁₀arylthio, C₁-C₁₀ alkyl, C₁-C₁₀ alkoxy, C₄-C₁₀ aryloxy, C₁-C₁₀ alkylcarbonyloxy, C₄-C₁₀ arylcarbonyloxy, C₃-C₈ cycloalkyl, C₃-C₈ cycloalkyloxy, C₂-C₁₀ alkenyl, C₂-C₁₀ alkynyl, C₄-C₁₀ aryl, carboxylate, aminocarbonyl, C₁-C₁₀ alkylcarbonyl, C₃-C₈ cycloalkylcarbonyl, C₃-C₈ heterocycloalkylcarbonyl, C₄-C₁₀ arylcarbonyl, C₄-C₁₀ aryloxycarbonyl, C₁-C₁₀ alkoxycarbonyl, C₃-C₈ cycloalkyloxycarbonyl, C₃-C₈ heterocycloalkyloxycarbonyl, C₄-C₁₀ aryloxycarbonyl, C₁-C₁₀ alkylsulfonyl, C₁-C₁₀ alkylamino, C₄-C₁₀ arylsulfonyl, C₃-C₈ heterocycloalkyl and C₄-C₁₀ heteroaryl.

Among the compounds according to the present invention, particularly preferred compounds are, but are not limited to, compounds listed in Table 1 below:

**Table 1**

| | | | |
|---|---|---|---|
| 1 | 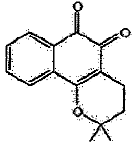 | C₁₅H₁₄O₃ | 242.27 |
| 2 | 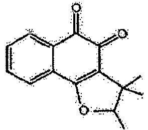 | C₁₅H₁₄O₃ | 242.27 |
| 3 | 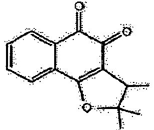 | C₁₅H₁₄O₃ | 242.27 |
| 4 | 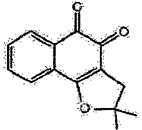 | C₁₄H₁₂O₃ | 228.24 |
| 5 | 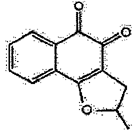 | C₁₃H₁₀O₃ | 214.22 |
| 6 | 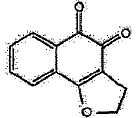 | C₁₂H₈O₃ | 200.19 |
| 7 | 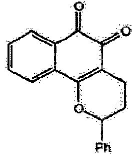 | C₁₉H₁₄O₃ | 290.31 |
| 8 | 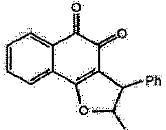 | C₁₉H₁₄O₃ | 290.31 |
| 9 | 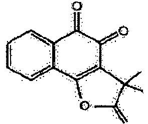 | C₁₅H₁₂O₃ | 240.25 |
| 10 | 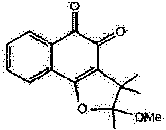 | C₁₅H₁₆O₄ | 272.30 |
| 11 | 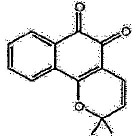 | C₁₅H₁₂O₃ | 240.25 |
| 12 | 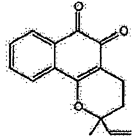 | C₁₆H₁₄O₃ | 254.28 |
| 13 | 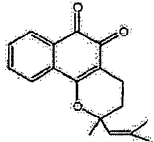 | C₁₈H₁₈O₃ | 282.33 |
| 14 | 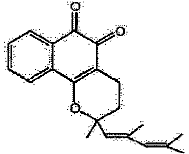 | C₂₁H₂₂O₃ | 322.40 |
| 15 | 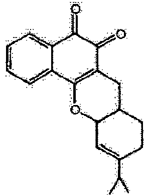 | C₂₁H₂₂O₃ | 322.40 |
| 16 | 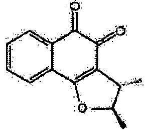 | C₁₄H₁₂O₃ | 228.24 |
| 17 | 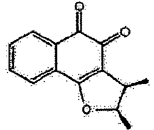 | C₁₄H₁₂O₃ | 228.24 |
| 18 | 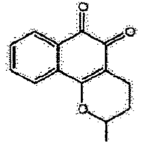 | C₁₄H₁₂O₃ | 228.24 |
| 19 | 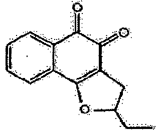 | C₁₄H₁₂O₃ | 228.24 |
| 20 | 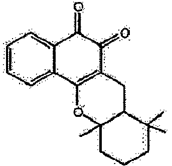 | C₂₀H₂₂O₃ | 310.39 |
| 21 | 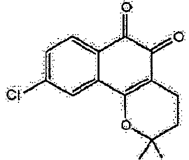 | C₁₅H₁₃ClO₃ | 276.71 |
| 22 | 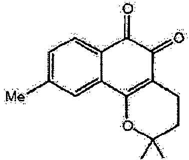 | C₁₆H₁₆O₃ | 256.30 |
| 23 | 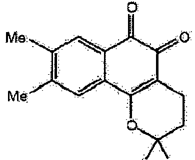 | C₁₇H₁₈O₅ | 302.32 |
| 24 | 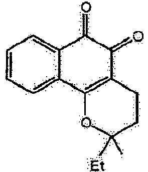 | C₁₆H₁₆O₃ | 256.30 |
| 25 | 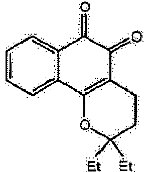 | C₁₇H₁₈O₃ | 270.32 |
| 26 | 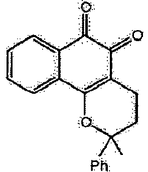 | C₂₀H₁₆O₃ | 304.34 |
| 27 | 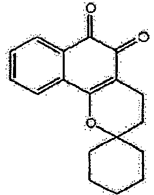 | C₁₈H₁₈O₃ | 282.33 |
| 28 | 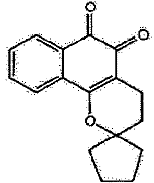 | C₁₇H₁₆O₃ | 268.31 |
| 29 | 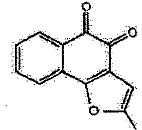 | C₁₃H₈O₃ | 212.20 |
| 30 | 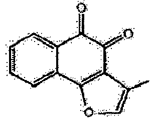 | C₁₃H₈O₃ | 212.20 |
| 31 | 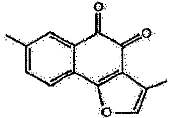 | C₁₄H₁₀O₃ | 226.23 |
| 32 | 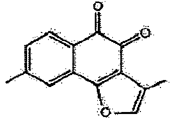 | C₁₄H₁₀O₃ | 226.23 |
| 33 | 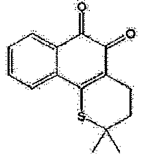 | C₁₅H₁₄O₂S | 258.34 |
| 34 | 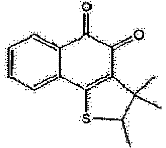 | C₁₅H₁₄O₂S | 258.34 |
| 35 | 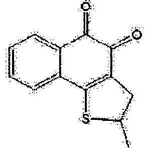 | C₁₃H₁₀O₂S | 230.28 |
| 36 | 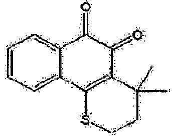 | C₁₅H₁₄O₂S | 258.34 |
| 37 | 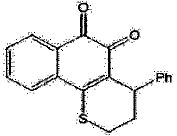 | C₁₉H₁₄O₂S | 306.38 |
| 38 | 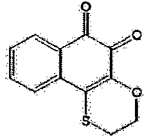 | C₁₂H₈O₃S | 232.26 |
| 39 | 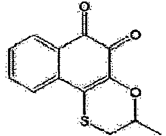 | C₁₃H₁₀O₃S | 246.28 |
| 40 | 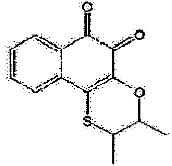 | C₁₄H₁₂O₃S | 260.31 |
| 41 | 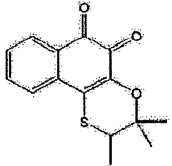 | C₁₅H₁₄O₃S | 274.34 |
| 42 | 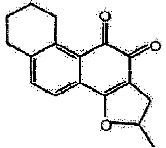 | C₁₇H₁₆O₃ | 268.31 |
| 43 | 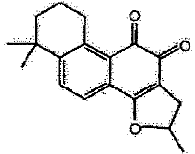 | C₁₉H₂₀O₃ | 296.36 |
| 44 | 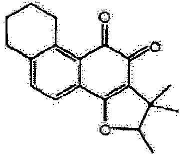 | C₁₉H₂₀O₃ | 296.36 |
| 45 | 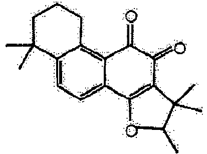 | C₂₁H₂₄O₃ | 324.41 |
| 46 | 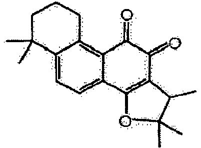 | C₂₁H₂₄O₃ | 324.41 |
| 47 | 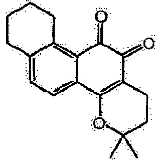 | C₁₉H₂₀O₃ | 296.36 |
| 48 | 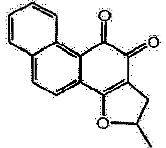 | C₁₇H₁₂O₃ | 264.28 |
| 49 | 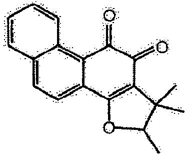 | C₁₉H₁₆O₃ | 292.33 |
| 50 | 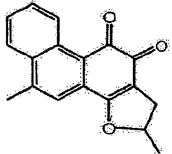 | C₁₈H₁₄O₃ | 278.30 |
| 51 | 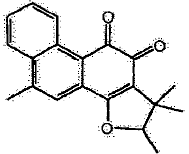 | C₂₀H₁₈O₃ | 306.36 |
| 52 | 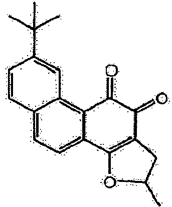 | C₂₁H₂₀O₃ | 320.38 |
| 53 | 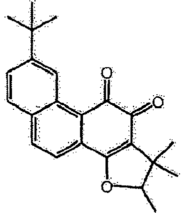 | C₂₃H₂₄O₃ | 348.43 |
| 54 | 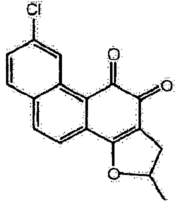 | C₁₇H₁₁ClO₃ | 298.72 |
| 55 | 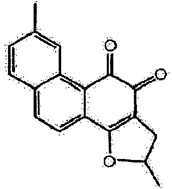 | C₁₈H₁₄O₃ | 278.30 |
| 56 | 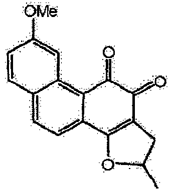 | C₁₈H₁₄O₄ | 294.30 |
| 57 | 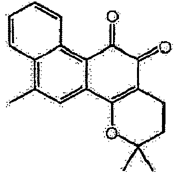 | C₂₀H₁₈O₃ | 306.36 |
| 58 | 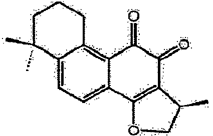 | C₁₈H₁₈O₃ | 282.33 |
| 59 | 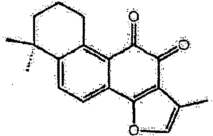 | C₁₈H₁₆O₃ | 280.33 |
| 60 | 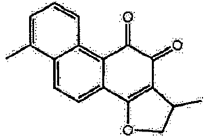 | C₁₈H₁₄O₃ | 278.33 |
| 61 | 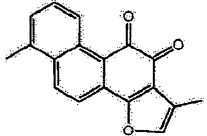 | C₁₈H₁₂O₃ | 276.33 |

The naphthoquinone-based compounds used in the composition of the present invention may be prepared by the methods disclosed in International Patent Publication WO 06/088315 and WO 06/020719, other known methods and/or various methods based on organic synthesis.

As used herein, the term "hearing loss" may include conductive and sensorineural hearing loss, preferably sensorineural hearing loss generated by disturbances of cochlea which functions to sense a sound or disturbances of acoustic or central nerves which transfer sound stimulus into brain. The hearing loss includes age-related presbycusis and ototoxic drug-related hearing loss.

The naphthoquinone-based compounds used in the composition of present invention exhibit an effect to recover auditory brainstem response threshold in animal models of age-related presbycusis and ototoxic drug-related hearing loss, and an effect to prevent hearing loss by inhibiting the apoptosis of auditory hair cells and HMGB1 (high-mobility group box-1) expression increase.

Accordingly, the composition of the present invention can be used as a pharmaceutical composition for treating or preventing hearing loss.

The pharmaceutical composition according to the present invention can be administered orally, e.g., ingestion or inhalation; or parenterally, e.g., injection, deposition, implantation or suppositories. The injection can be, for example, intravenous, intradermal, subcutaneous, intramuscular or intraperitoneal. Depending on the route of administration, the pharmaceutical composition of the present invention may be formulated as tablets, capsules, granules, fine subtilae, powders, sublingual tablets, suppositories, ointments, injection solutions, emulsions, suspensions, syrups, aerosols, etc. The above various forms of the pharmaceutical composition of the present invention can be prepared in a manner well known in the art using a pharmaceutically acceptable carrier(s) which are usually used for each form. Examples of the pharmaceutically acceptable carriers include excipient, binder, disintegrator, lubricant, preservative, antioxidant, isotonic agent, buffer, coating agent, sweetening agent, dissolvent, base, dispersing agent, wetting agent, suspending agent, stabilizer, colorant, etc.

The pharmaceutical composition of the present invention contains about 0.01 to 100 wt% of the naphthoquinone-based compounds depending on the form thereof.

The specific dosage of the present pharmaceutical composition can be varied with species of mammals including a human-being, body weight, gender, severity of disease, judgment of doctor, etc. It is preferable that 0.001 to 500 mg of the active ingredient is administered per kg of body weight a day for oral use, while 0.001 to 1000 mg of the active ingredient is administered per kg of body weight a day for parenteral use. The total daily dosage can be administered once or over several times depending on the severity of disease, judgment of doctor, etc.

In accordance with another aspect of the present invention, the composition of the present invention can be used as a functional food for ameliorating or preventing hearing loss.

There is no particular limit to the kinds of the functional food according to the present invention. It can be oral preparation such as powders, granules, tablets, capsules, suspensions, emulsions, syrups, etc., or ordinary food such as candies, snacks, gums, ice creams, noodles, breads, drinks, etc. to which the active ingredient is added.

The functional food according to the present invention can be prepared in a manner well known in the art using a sitologically acceptable carrier(s) such as filler, extender, binder, wetting agent, disintegrator, sweetening agent, flavoring agent, preservative, surfactant, lubricant, excipient, etc. depending on the form thereof,

The functional food of the present invention contains about 0.01 to 100 wt% of the naphthoquinone-based compounds depending on the form thereof.

### [Advantageous Effects]

The composition comprising naphthoquinone-based compounds according to the present invention is useful for treating or preventing hearing loss selected from age-related presbycusis and ototoxic drug-related hearing loss.

### [Description of Drawings]

Fig. 1 is a graph showing the auditory brainstem response thresholds of 2- and 12-month-old lab animals.
Fig. 2 is a graph showing the variations of the auditory brainstem response thresholds between the experiment groups of 15-month-old lab animals.
Fig. 3 is a graph showing the variations of the auditory brainstem response thresholds between the experiment groups of 18-month-old lab animals.
Fig. 4 is a graph showing the variations of the auditory brainstem response thresholds between the experiment groups of 21-month-old lab animals.
Fig. 5 is a graph showing the variations of the auditory brainstem response thresholds between the experiment groups of 24-month-old lab animals.
Fig. 6 shows an inhibiting effect of β-lapachone (βL) against the apoptosis of auditory hair cells due to aging.
Fig. 7 shows an inhibiting effect of β-lapachone (βL) against HMGB 1 expression increase due to aging.
Fig. 8 is a graph showing the variations of the auditory brainstem response thresholds between the experiment groups of 6-week-old lab animals.
Fig. 9 is a graph showing an inhibiting effect of β-lapachone (βL) against the apoptosis of auditory hair cells by cisplatin (CDDP).
Fig. 10 shows an inhibiting effect of β-lapachone (βL) against the expression increase of inflammation mediators by cisplatin (CDDP).
Fig. 11 is a graph showing an inhibiting effect of β-lapachone (βL) against the release increase of HSP60 by cisplatin (CDDP) in mice.
Fig. 12 is a graph showing an inhibiting effect of β-lapachone (βL) against the release increase of HSP60 by cisplatin (CDDP) in HEI-OC1 cells.
Fig. 13 is a graph showing the function of HSP60 in the apoptosis of auditory hair cells by cisplatin (CDDP).
Fig. 14 is a graph showing an inhibiting effect of β-lapachone (βL) against the release increase of HMGB 1 by cisplatin (CDDP).

### [Best Mode]

The present invention is further illustrated by the following examples, which are not to be construed to limit the scope of the invention.

### Experimental Example 1: Animal Model with Age-related Presbycusis

### Experimental Example 1-1: Preparation of Animal Model

C57BL/6 mice which are well known as a age-related hearing loss model were used for this experiment. 12-month-old C57BL/6 mice were directly purchased from Central Lab. Animal Inc. (Seoul, Korea), and bred up to 24 months of age with measuring hearing loss every 3 months. All mice used in this experiment were bred in an axenic (germfree) animal facility maintaining constant temperature (22 to 26 °C) and humidity (55 to 60%).

All experiments were conducted for groups divided into a control group freely providing normal feed, caloric restriction (CR) group limiting dietary to 70%, and βL group providing feed containing 0.06% β-lapachone (compound 1).

### Experimental Example 1-2: Variation of Auditory Brainstem Response (ABR) Threshold

In order to evaluate auditory brainstem response, all lab animals were intramuscularly injected with a mixture of xylazine (5 mg/kg) and ketamine for anesthesia and the variation of auditory brainstem response threshold was measured using a TDT system (Tucker-Davis Technologies, FL, USA) in a soundproof room.

An insertable ear tip (3.5 mm, Nicolet Biomedical, Inc.) was placed in the external auditory meatus, electrodes were placed at scalp vertex (active electrode), the mastoid region of the ear of interest (reference electrode) and the mastoid region of the opposite ear (ground electrode), and the threshold of ABS was measured by starting with 90 dB HL intensity and then sequentially decreasing the intensity by 10 dB at 4, 8, 16 and 32 kHz.

There was no arousal response and death due to respiratory depression during the ABR test. The comparison of the ABR thresholds between groups was conducted by one-way analysis of variance (ANOVA) and P value of 0.05 or less was regarded as being significant.

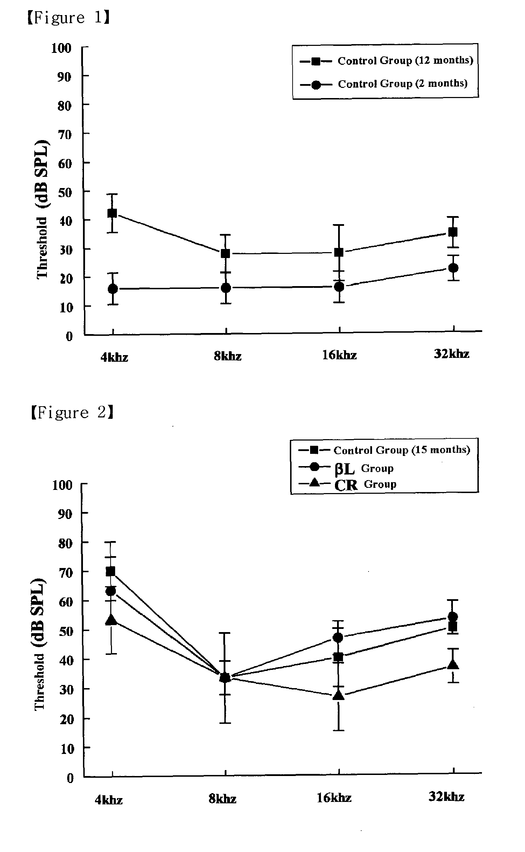

As shown in Fig. 1, the ABR thresholds of 12-month-old lab animals significantly increased by 10 dB SPL or higher, as compared to those of 2-month-old lab animals.

Then, the variations of the ABS thresholds were measured for three groups of 15-month-old lab animals. As shown in Fig. 2, the ABR thresholds of the control group were 70±10, 33.33±5.77, 40±10 and 50±10 dB SPL at 4, 8, 16 and 32 kHz, respectively; the βL group, 63.33±11.55, 33.33±15.28, 46.67±5.77 and 53.33±5.77 dB SPL, respectively; and CR group, 53.33±11.55, 33.33±5.77, 26.67±11.55 and 36.67±5.77 dB SPL, respectively. The results exhibited statistically significant recovery only at 32 kHz in comparison with the control group (*p*<0.05).

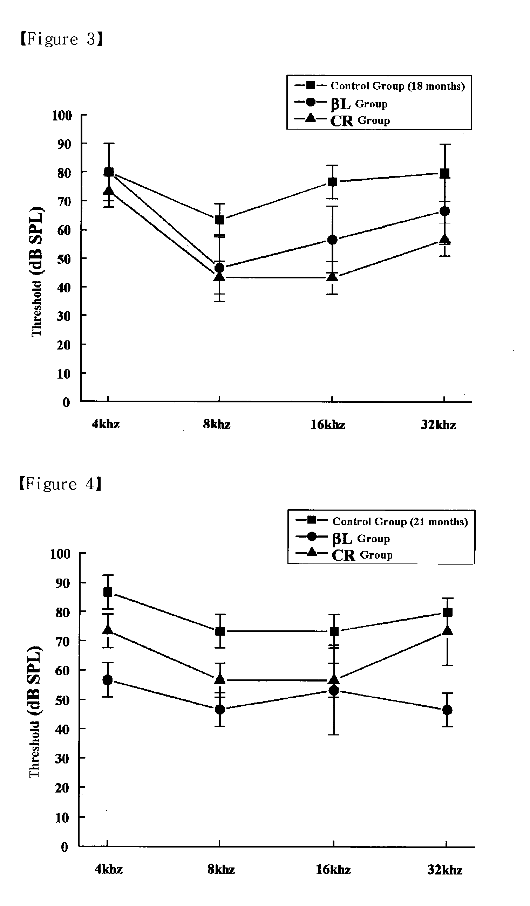

The variations of the ABR thresholds for three groups of 18-month-old lab animals were measured. As shown in Fig. 3, the ABR thresholds of the control group were 80±10, 63.33±5.77, 76.67±5.77 and 80±10 dB SPL at 4, 8, 16 and 32 kHz, respectively; the βL group, 80±0, 46.67±11.55, 56.67±11.55 and 66.67±11.55 dB SPL, respectively; and CR group, 73.33±5.77, 43.33±5.77, 43.33±5.77 and 56.67±5.77 dB SPL, respectively. The results exhibited statistically significant recovery at 8 to 32 kHz in comparison with the control group (*p*<0.05).

Next, the variations of the ABR thresholds for three groups of 21-month-old lab animals were measured. As shown in Fig. 4, the ABR thresholds of the control group were 86.67±5.77, 73.33±5.77, 73.33±5.77 and 80±0 dB SPL at 4, 8, 16 and 32 kHz, respectively, and the βL group, 56.67±5.77, 46.67±5.77, 53.33±15.28 and 46.67±5.77 dB SPL, respectively. The results exhibited statistically significant recovery at regions except for 16 kHz in comparison with the control group (*p*<0.05). For the CR group, the ABR thresholds were 73.33±5.77, 56.67±5.77, 56.67±5.77 and 73.33±11.55 dB SPL at 4, 8, 16 and 32 kHz, respectively. The results exhibited statistically significant recovery at regions except for 32 kHz in comparison with the control group (*p*<0.05).

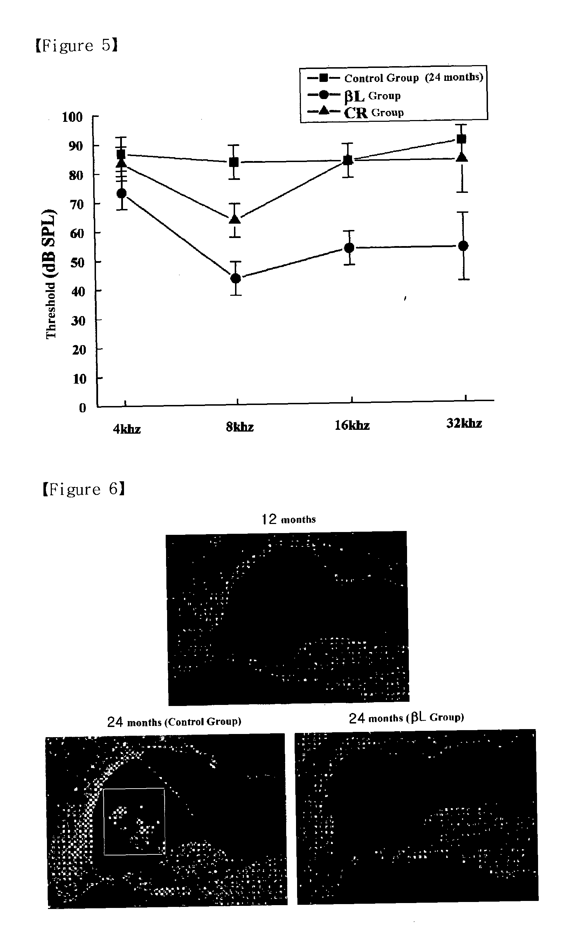

The variations of the ABR thresholds for three groups of 24-month-old lab animals were measured. As shown in Fig. 5, the ABR thresholds of the control group were 86.67±5.77, 83.33±5.77, 83.33±5.77 and 90±0 dB SPL at 4, 8, 16 and 32 kHz, respectively, and the βL group, 73.33±5.77, 43.33±5.77, 53.33±5.77 and 53.33±11.55 dB SPL, respectively. The results exhibited statistically significant recovery at all regions in comparison with the control group (*p*<0.05), particlularly recovery no less than 30 dB SPL at 8 to 32 kHz except for 4 kHz. Meanwhile, for the CR group, the ABR thresholds were 83.33±5.77, 63.33±5.77, 83.33±5.77 and 83.33±11.55 dB SPL at 4, 8, 16 and 32 kHz, respectively. The results exhibited statistically significant recovery only at 8 kHz in comparison with the control group (*p*<0.05).

### Experimental Example 1-3: Identification of Hair Cell and Mitochondria Damage

In order to identify damage of hair cells, the inner ears of the lab animals were enucleated and subject to terminal deoxynucleotidyl transferase-mediated dUTP nick end-labeling (TUNEL) from the top of cochlea to middle circular part thereof, followed by analyzing TUNEL positive cells usig a fluorescence microscope.

As shown in Fig. 6, the control group of 24-month-old lab animals exhibited apoptosis in outer and inner hair cells and surrounding cells, while the βL group thereof significantly inhibited the age-related apoptosis in the whole inner ear.

Also, in order to identify inhibition of age-related mitochondria damage by β-lapachone, the shape variations of mitochondria were detected for three groups of 24-month-old lab animals using a transmission electron microscope (TEM).

As a result, the control group and the CR group of the 24-month-old lab animals exhibited damage or loss of even more mitochondria as compared to 2-month-old lab animals, while the βL group thereof exhibited the number and shape of mitochondria similar to the 2-month-old lab animals.

The results clearly show that β-lapachone inhibits the damage of hair cells and mitochondria due to aging.

### Experimental Example 1-4: Analysis of HMGB 1 Expression

In order to identify an effect of β-lapachone against the expression of HMGB 1 which is known to be important for the damage and shape variation of mitochondria, a tissue staining was conducted.

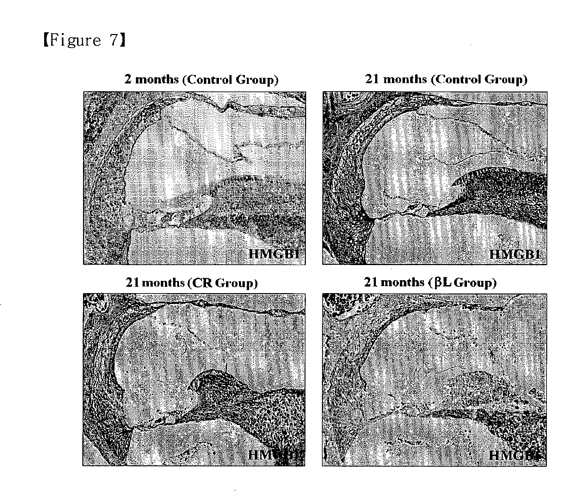

As shown in Fig. 7, the control group and the CR group of 21-month-old lab animals exhibited increased expression of HMGB1 throughout the whole inner ear as compared to 2-month-old lab animals, while the βL group thereof exhibited significantly decreased expression of HMGB1 as compared to the other groups.

Thus, it has been confirmed that β-lapachone inhibits the increase of HMGB1 expression due to aging to inhibit the apoptosis of the cells in the inner ear tissue and variation of the number and shape of mitochondria, thereby effectively protecting hearing loss due to aging.

### Experimental Example 2: Animal Model with Ototoxic Drug (Cisplatin)-related Hearing Loss

### Experimental Example 2-1: Preparation of Animal Model

6-week-old C57BL/6 mice were bred in an axenic (germfree) animal facility maintaining constant temperature (22 to 26 °C) and humidity (55 to 60%).

All experiments were conducted for groups divided into a control group injected with PBS only, a CDDP group intraperitoneally injected with cisplatin (4 mg/kg/day) for 4 days, a group injected with β-lapachone (10 mg/kg/day, 20 mg/kg/day and 40 mg/kg/day) and cisplatin for 4 days, and a βL group injected with β-lapachone (40 mg/kg) only.

### Experimental Example 2-2: Variation of Auditory Brainstem Response (ABR) Threshold

The ABR test was conducted by the same method as Experimental Example 1-2 to measure thresholds before and after the injection of each drug. The comparison of the ABR thresholds between groups was conducted by one-way analysis of variance (ANOVA) and P value of 0.05 or less was regarded as being significant.

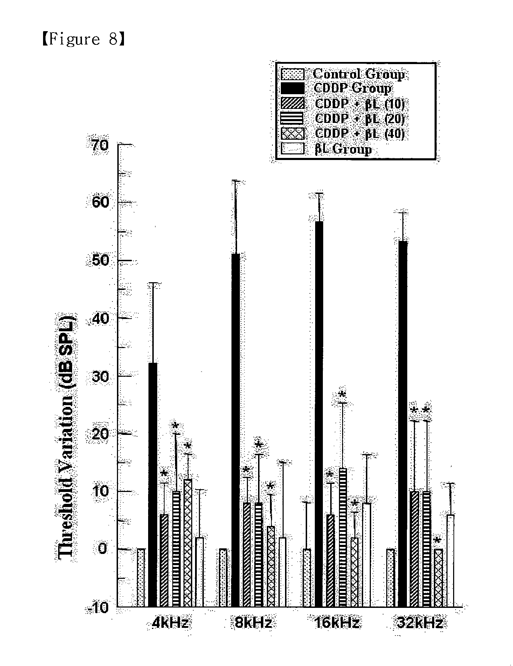

As shown in Fig. 8, the CDDP group injected with cisplatin only exhibited increase of the ABR thresholds by 30 dB SPL or higher on average at all regions, while the group injected with both β-lapachone and cisplatin exhibited statistically significant recovery at all regions, as compared to the CDDP group (*p*<0.05). Also, the control group did not exhibit distinct variations of the ABR thresholds at all regions, and the βL group exhibited only slight variations of the ABR thresholds at all regions, similar to the control group.

### Experimental Example 2-3: Identification of Hair Cell Damage

In order to identify whether β-lapachone protects hearing loss due to cisplatin by directly inhibiting apoptosis of auditory hair cells, HEI-OC1, which is an auditory hair cell line, was treated with β-lapachone in various concentrations 30 minutes before treating with cisplatin, and cell viability rates were measured by the MTT method.

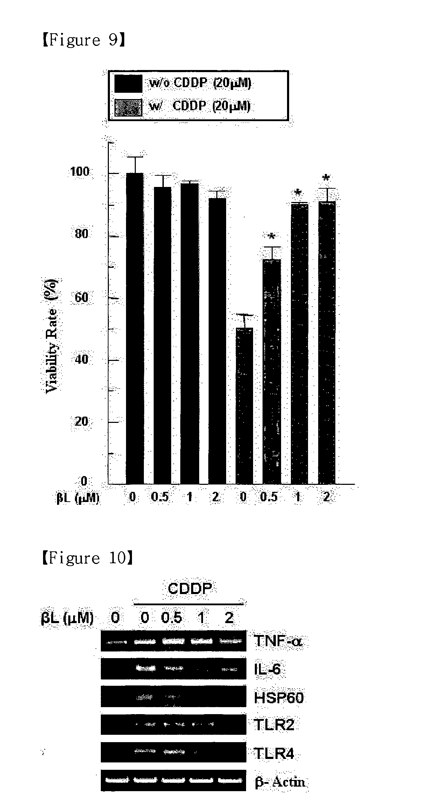

As shown in Fig. 9, the single treatment of β-lapachone did not affect the viability rates of HEI-OCl in a concentration of 2 µM or less, and β-lapachone protected the apoptosis by cisplatin in a concentration-dependent manner (*p*<0.05).

Also, the explants of the Corti's organ showed that the shape and arrangement of the outer and inner hair cells were changed upon treating with cisplatin, while the group treating with both β-lapachone and cisplatin maintained the shape and arrangement of the auditory hair cells similar to the control group.

### Experimental Example 2-4: Analysis of Inflammation Mediator Expression

The expression of inflammation mediators which are known as an important factor in hearing loss due to cisplatin was identified by the PCR method. β-lapachone was treated in various concentrations 30 minutes before treating with cisplatin, and the expression of inflammation mediators by cisplatin was measured by the PCR method.

As shown in Fig. 10, HEI-OC1 cells treated with cisplatin exhibited increased expression of inflammation-inducing cytokines such as TNF-α, IL-6 and HSP60 and inflammation mediators such as TLR2 and TLR4, and the expression was inhibited by β-lapachone in a concentration-dependent manner.

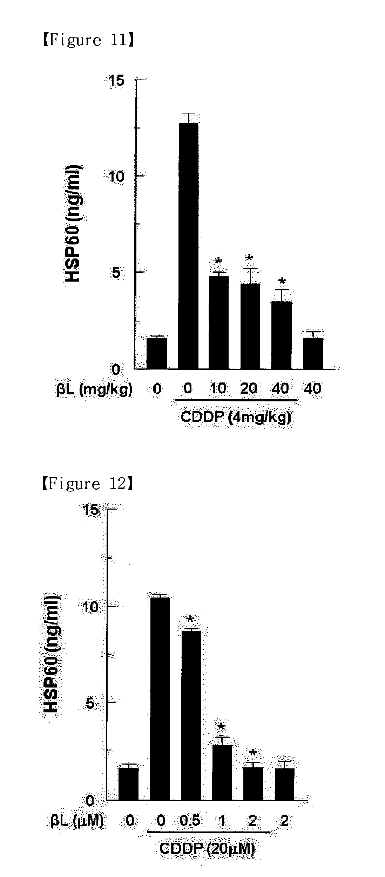

It is generally known that HSP60 in mitochondria is released outside of the cells to bind to TLR2 or TLR4, thereby inducing strong inflammation. Accordingly, the release amounts of HSP60 in mice and HEI-OC1 cells were measured by the ELISA method. As shown in Figs. 11 and 12, mice and HEI-OC1 cells treated with cisplatin exhibited significantly increased release amounts of HSP60 (*p*<0.05). However, mice and HEI-OC1 cells treated with both β-lapachone and cisplatin exhibited decreased release amounts of HSP60 in a β-lapachone concentration-dependent manner.

Also, location variation of HSP60 was identified by cell staining. In normal cells (the control group), HSP60 was almost expressed in mitochondria, while in cells treated with cisplatin, HSP60 was almost expressed in cytoplasm. In cells treated with both β-lapachone and cisplatin, HSP60 was expressed in only mitochondria like the normal cells.

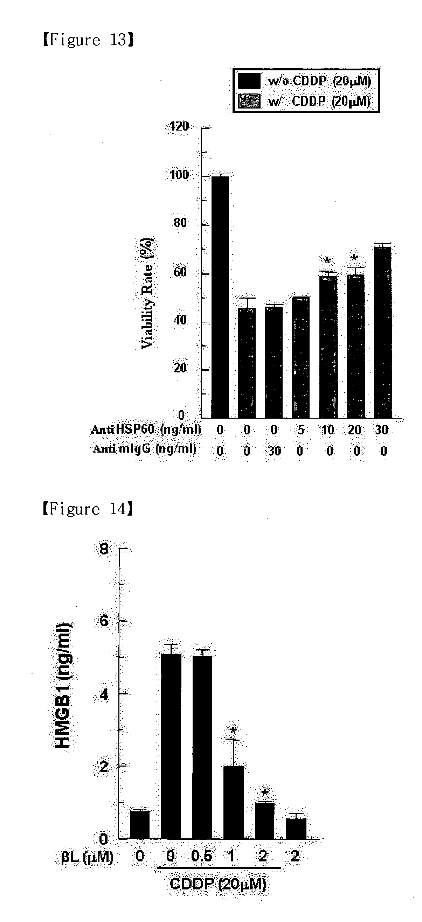

Next, in order to identify whether HSP60 directly affects the death of auditory cells by cisplatin, a HSP60 antibody was treated together with cisplatin and cell viability rates were measured by the MTT method. As shown in Fig. 13, the death of HEI-OC1 cells by cisplatin was inhibited by the treatment of the HSP60 antibody (*p*<0.05).

HMGB1 is, together with HSP60, known as an important mediator for the activation of TLRs and the resulting inflammation. Accordingly, the extracellular release of HMGB1 was identified by the ELISA method. As shown in Fig. 14, HEI-OC1 cells treated with cisplatin exhibited extracellular release of excessive amounts of HMGB1, and the release amounts decreased in a β-lapachone concentration-dependent manner (*p*<0.05).

Accordingly, it has been confirmed that β-lapachone inhibits the expression of several inflammation mediators to effectively protect the death of auditor hair cells and hearing loss by cisplatin.

## Claims

1. A composition comprising a compound of Formula 1 or 2, or a pharmaceutically acceptable salt, solvate or isomer thereof for use in treating or preventing hearing loss selected from age-related presbycusis and ototoxic drug-related hearing loss: 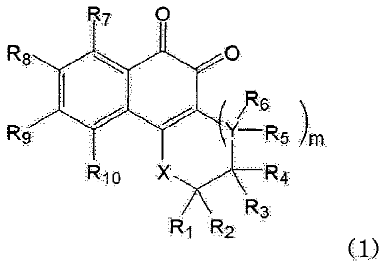 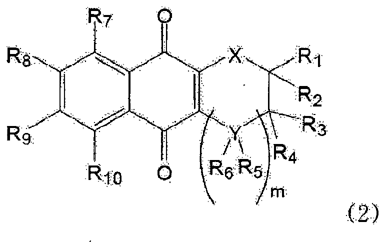 wherein,
R₁ to R₆ are each independently selected from the group consisting of hydrogen, hydroxy, halogen, amino, substituted or unsubstituted C₁-C₁₀ alkylamino, substituted or unsubstituted C₁-C₁₀ dialkylamino, substituted or unsubstituted C₁-C₁₀ alkyl, substituted or unsubstituted C₂-C₁₀ alkenyl, substituted or unsubstituted C₂-C₁₀ alkynyl, substituted or unsubstituted C₁-C₁₀ alkoxy, substituted or unsubstituted C₁-C₁₀ alkoxycarbonyl, substituted or unsubstituted C₁-C₁₀ acyl, substituted or unsubstituted C₃-C₈ cycloalkyl, substituted or unsubstituted C₃-C₈ heterocycloalkyl, substituted or unsubstituted C₄-C₁₀ aryl, substituted or unsubstituted C₄-C₁₀ heteroaryl, and substituted or unsubstituted -(CH₂)ₙ-aryl, or two substituents of R₁ to R₆ may be taken together to form a double bond or a substituted or unsubstituted C₃-C₆ cyclic structure which may be saturated or partially or completely unsaturated; the substituent being at least one selected from the group consisting of hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₁-C₁₀ alkylamino, C₃-C₈ cycloalkyl, C₃-C₈ heterocycloalkyl, C₄-C₁₀ aryl, and C₄-C₁₀ heteroaryl;
R₇ to R₁₀ are each independently selected from the group consisting of hydrogen, hydroxy, halogen, amino, substituted or unsubstituted C₁-C₁₀ alkylamino, substituted or unsubstituted C₁-C₁₀ dialkylamino, substituted or unsubstituted C₁-C₁₀ alkyl, substituted or unsubstituted C₂-C₁₀ alkenyl, substituted or unsubstituted C₂-C₁₀ alkynyl, substituted or unsubstituted C₁-C₁₀ alkoxy, substituted or unsubstituted C₁-C₁₀ alkoxycarbonyl, substituted or unsubstituted C₁-C₁₀ acyl, substituted or unsubstituted C₃-C₈ cycloalkyl, substituted or unsubstituted C₃-C₈ heterocycloalkyl, substituted or unsubstituted C₄-C₁₀ aryl, substituted or unsubstituted C₄-C₁₀ heteroaryl, and substituted or unsubstituted -(CH₂)ₙ-aryl, or two substituents of R₇ to R₁₀ may be taken together to form a substituted or unsubstituted C₃-C₆ cyclic structure which may be saturated or partially or completely unsaturated; the substituent being at least one selected from the group consisting of hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₁-C₁₀ alkylamino, C₃-C₈ cycloalkyl, C₃-C₈ heterocycloalkyl, C₄-C₁₀ aryl, and C₄-C₁₀ heteroaryl;
X is O, S or NR', with R' being hydrogen or C₁-C₆ alkyl;
Y is C, S, N or O, with the proviso that when Y is S or O, R₅ and R₆ are not any substituent, and when Y is N, R₅ is hydrogen or C₁-C₆ alkyl and R₆ is not any substituent;
m is 0 or 1, with the proviso that when m is 0, carbon atoms adjacent to m form a cyclic structure via a direct bond; and
n is an integer of 0 to 10.

2. The composition for use according to claim 1, wherein X is O or S, and Y is C or O.

3. The composition for use according to claim 1, wherein R₁ to R₆ are each independently selected from the group consisting of hydrogen, hydroxy, halogen, substituted or unsubstituted C₁-C₁₀ alkyl, substituted or unsubstituted C₂-C₁₀ alkenyl, substituted or unsubstituted C₁-C₁₀ alkoxy, and -(CH₂)ₙ-phenyl, or R₁ and R₄ or R₂ and R₃ is taken together to form a double bond or a C₄-C₆ cyclic structure, the substituent being C₁-C₁₀ alkyl.

4. The composition for use according to claim 1, wherein R₇ to R₁₀ are each independently selected from the group consisting of hydrogen, hydroxy, halogen, substituted or unsubstituted C₁-C₁₀ alkyl, and substituted or unsubstituted C₁-C₁₀ alkoxy.

5. The composition for use according to claim 1, wherein the compound of Formula 1 or 2 is a compound of Formula 1-1, 2-1, 1-2 or 2-2: 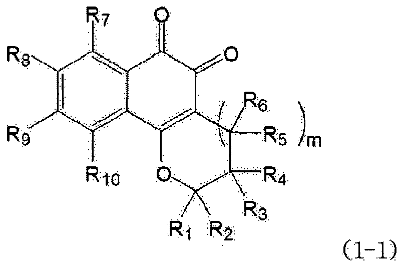 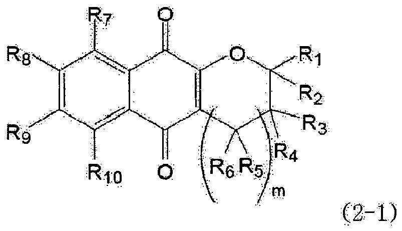 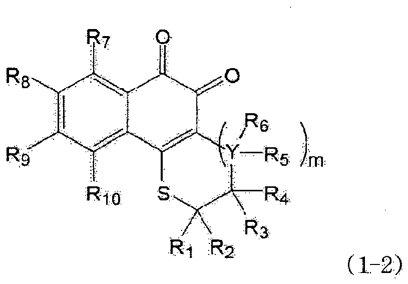 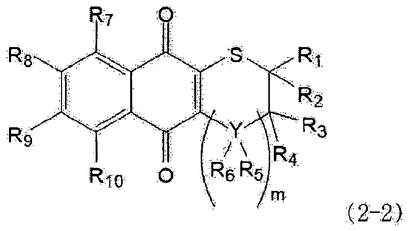 wherein, R₁ to R₁₀, Y and m have the same meanings as defined in claim 1.

6. The composition for use according to claim 1, wherein the compound of Formula 1 or 2 is a compound of Formula 1-3 or 2-3: 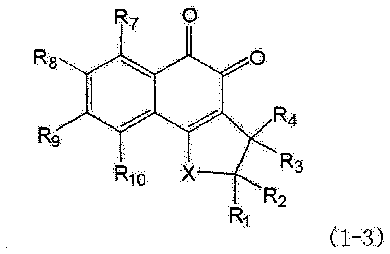 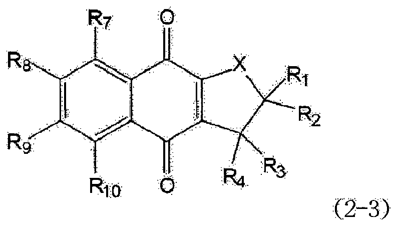 wherein, R₁ to R₁₀, and X have the same meanings as defined in claim 1.

7. The composition for use according to claim 1, wherein the compound of Formula 1 or 2 is a compound of Formula 1-4 or 2-4: 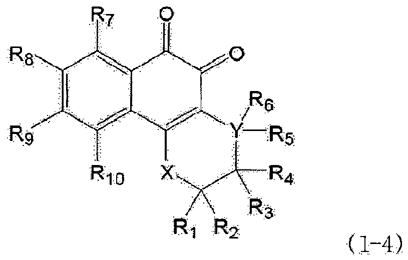 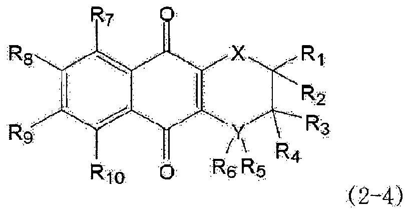 wherein, R₁ to R₁₀, X and Y have the same meanings as defined in claim 1.

8. The composition for use according to claim 1, wherein the compound of Formula 1 or 2 is a compound of Formula 1-5, 1-6, 2-5 or 2-6: 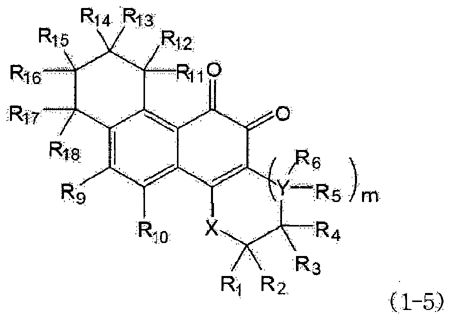 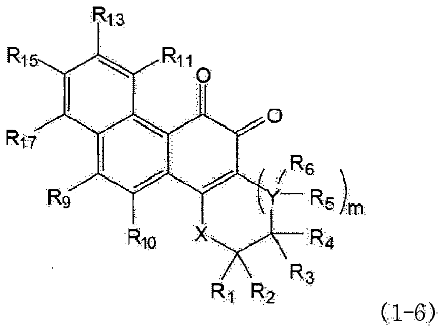 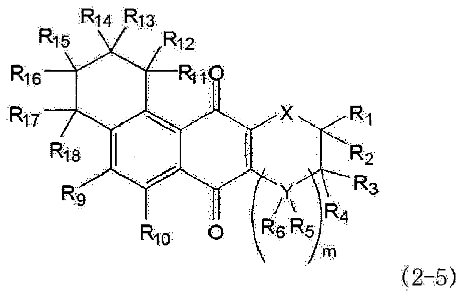 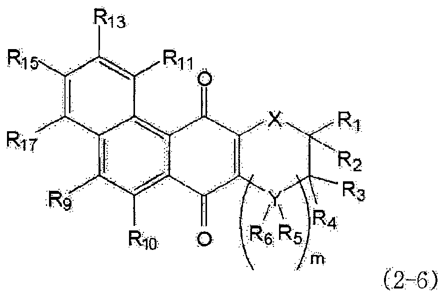 wherein,
R₁ to R₁₀, X, Y and m have the same meanings as defined in claim 1, and
R₁₁ to R₁₈ are each independently selected from the group consisting of hydrogen, hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₁-C₁₀ alkylamino, C₃-C₈ cycloalkyl, C₃-C₈ heterocycloalkyl, C₄-C₁₀ aryl, and C₄-C₁₀ heteroaryl.

9. The composition for use according to claim 8, wherein R₁₁ to R₁₈ are each independently selected from the group consisting of hydrogen, hydroxy, halogen, C₁-C₁₀ alkyl, C₂-C₁₀ alkenyl, C₂-C₁₀ alkynyl, C₁-C₁₀ alkoxy, C₃-C₈ cycloalkyl, and phenyl.

10. The composition for use according to claim 7, wherein
R₁ and R₂ are C₁-C₁₀ alkyl,
R₃ to R₁₀ are hydrogen,
X is O, and
Y is C.

11. The composition for use according to claim 1, wherein the hearing loss is age-related presbycusis.

12. The composition for use according to claim 1, wherein the hearing loss is ototoxic drug-related hearing loss.

13. The composition for use according to any one of claims 1 to 12, which is a pharmaceutical composition comprising the compound of Formula 1 or 2, or a pharmaceutically acceptable salt, solvate or isomer thereof together with a pharmaceutically acceptable carrier.

14. The composition for use according to any one of claims 1 to 12, which is a functional food comprising the compound of Formula 1 or 2, or a pharmaceutically acceptable salt, solvate or isomer thereof together with a sitologically acceptable carrier.

15. The composition for use according to claim 1, wherein the compound is a compound of formula:

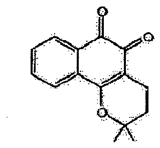

## Patentansprüche

1. Zusammensetzung, umfassend eine Verbindung der Formel 1 oder 2 oder ein pharmazeutisch akzeptables Salz, Solvat oder Isomer davon, zur Verwendung bei der Behandlung oder Prävention von Hörverlust, ausgewählt aus altersbedingter Presbyakusis und ototoxischem, arzneimittelbedingtem Hörverlust: 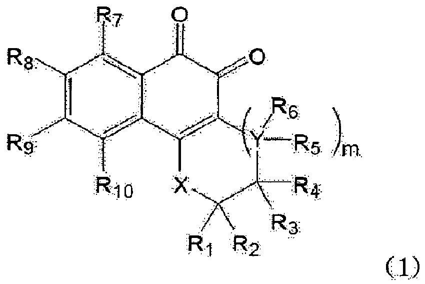 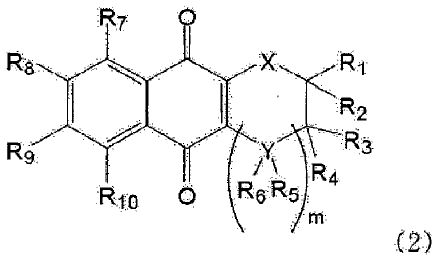
wobei R₁ bis R₆ jeweils unabhängig ausgewählt sind aus der Gruppe bestehend aus Wasserstoff, Hydroxy, Halogen, Amino, substituiertem oder unsubstituiertem C₁-C₁₀ Alkylamino, substituiertem oder unsubstituiertem C₁-C₁₀ Dialkylamino, substituiertem oder unsubstituiertem C₁-C₁₀ Alkyl, substituiertem oder unsubstituiertem C₂-C₁₀ Alkenyl, substituiertem oder unsubstituiertem C₂-C₁₀ Alkinyl, substituiertem oder unsubstituiertem C₁-C₁₀ Alkoxy, substituiertem oder unsubstituiertem C₁-C₁₀ Alkoxycarbonyl, substituiertem oder unsubstituiertem C₁-C₁₀ Acyl, substituiertem oder unsubstituiertem C₃-C₈ Cycloalkyl, substituiertem oder unsubstituiertem C₃-C₈ Heterocycloalkyl, substituiertem oder unsubstituiertem C₄-C₁₀ Aryl, substituiertem oder unsubstituiertem C₄-C₁₀ Heteroaryl und substituiertem oder unsubstituiertem -(CH₂)ₙ-Aryl, oder zwei Substituenten von R₁ bis R₆ können zusammengenommen werden, um eine Doppelbindung oder eine substituierte oder unsubstituierte C₃-C₆ zyklische Struktur, die gesättigt oder teilweise oder vollständig ungesättigt sein kann, zu bilden; wobei der Substituent mindestens einer ist, ausgewählt aus der Gruppe bestehend aus Hydroxy, Halogen, C₁-C₁₀ Alkyl, C₂-C₁₀ Alkenyl, C₂-C₁₀ Alkinyl, C₁-C₁₀ Alkoxy, C₁-C₁₀ Alkoxycarbonyl, C₁-C₁₀ Alkylamino, C₃-C₈ Cycloalkyl, C₃-C₈ Heterocycloalkyl, C₄-C₁₀ Aryl und C₄-C10 Heteroaryl;
R₇ bis R₁₀ jeweils unabhängig ausgewählt sind aus der Gruppe bestehend aus Wasserstoff, Hydroxy, Halogen, Amino, substituiertem oder unsubstituiertem C₁-C₁₀ Alkylamino, substituiertem oder unsubstituiertem C₁-C₁₀ Dialkylamino, substituiertem oder unsubstituiertem C₁-C₁₀ Alkyl, substituiertem oder unsubstituiertem C₂-C₁₀ Alkenyl, substituiertem oder unsubstituiertem C₂-C₁₀ Alkinyl, substituiertem oder unsubstituiertem C₁-C₁₀ Alkoxy, substituiertem oder unsubstituiertem C₁-C₁₀ Alkoxycarbonyl, substituiertem oder unsubstituiertem C₁-C₁₀ Acyl, substituiertem oder unsubstituiertem C₃-C₈ Cycloalkyl, substituiertem oder unsubstituiertem C₃-C₈ Heterocycloalkyl, substituiertem oder unsubstituiertem C₄-C₁₀ Aryl, substituiertem oder unsubstituiertem C₄-C₁₀ Heteroaryl, und substituiertem oder unsubstituiertem -(CH₂)ₙ-Aryl, oder zwei Substituenten von R₇ bis R₁₀ können zusammengenommen werden, um eine substituierte oder unsubstituierte C₃-C₆ zyklische Struktur, die gesättigt oder teilweise oder vollständig ungesättigt sein kann, zu bilden; wobei der Substituent mindestens einer ist, ausgewählt aus der Gruppe, bestehend aus Hydroxy, Halogen, C₁-C₁₀ Alkyl, C₂-C₁₀ Alkenyl, C₂-C₁₀ Alkinyl, C₁-C₁₀ Alkoxy, C₁-C₁₀ Alkoxycarbonyl, C₁-C₁₀ Alkylamino, C₃-C₈ Cycloalkyl, C₃-C₈ Heterocycloalkyl, C₄-C₁₀ Aryl und C₄-C₁₀ Heteroaryl;
X gleich O, S oder NR' ist, wobei R' Wasserstoff oder C₁-C₆ Alkyl ist;
Y gleich C, S, N oder O ist, unter der Voraussetzung, dass, wenn Y gleich S oder O ist, R₅ und R₆ keine Substituenten sind, und wenn Y gleich N ist, R₅ Wasserstoff oder C₁-C₆ Alkyl ist, und R₆ kein Substituent ist;
m gleich 0 oder 1 ist, unter der Voraussetzung, dass, wenn m 0 ist, Kohlenstoffatome angrenzend an m eine zyklische Struktur über eine direkte Bindung bilden; und
n eine ganze Zahl von 0 bis 10 ist.

2. Zusammensetzung zur Verwendung nach Anspruch 1, wobei X gleich O oder S ist und Y gleich C oder O ist.

3. Zusammensetzung zur Verwendung nach Anspruch 1, wobei R₁ bis R₆ jeweils unabhängig ausgewählt sind aus der Gruppe bestehend aus Wasserstoff, Hydroxy, Halogen, substituiertem oder unsubstituiertem C₁-C₁₀ Alkyl, substituiertem oder unsubstituiertem C₂-C₁₀ Alkenyl, substituiertem oder unsubstituiertem C₁-C₁₀ Alkoxy und -(CH₂)ₙ-Phenyl, oder R₁ und R₄ oder R₂ und R₃ zusammengenommen sind, um eine Doppelbindung oder eine C₄-C₆ zyklische Struktur zu bilden, wobei der Substituent C₁-C₁₀ Alkyl ist.

4. Zusammensetzung zur Verwendung nach Anspruch 1, wobei R₇ bis R₁₀ jeweils unabhängig ausgewählt sind aus der Gruppe bestehend aus Wasserstoff, Hydroxy, Halogen, substituiertem oder unsubstituiertem C₁-C₁₀ Alkyl und substituiertem oder unsubstituiertem C₁-C₁₀ Alkoxy.

5. Zusammensetzung zur Verwendung nach Anspruch 1, wobei die Verbindung von Formel 1 oder 2 eine Verbindung der Formel 1-1, 2-1, 1-2 oder 2-2 ist: 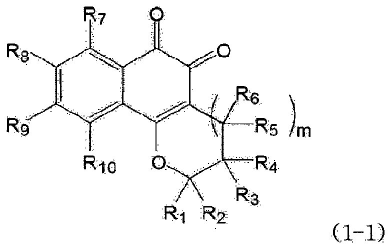 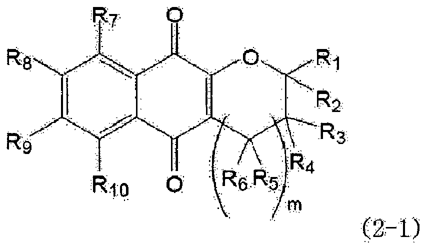 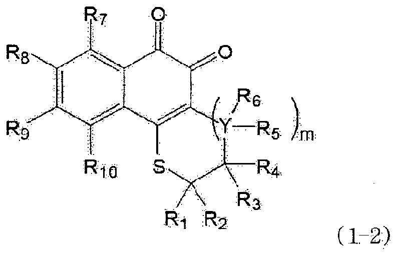 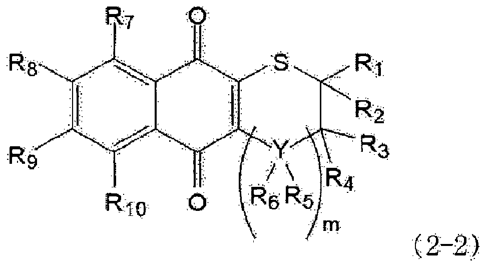 wobei R₁ bis R₁₀, Y und m dieselben Bedeutungen aufweisen wie in Anspruch 1 definiert.

6. Zusammensetzung zur Verwendung nach Anspruch 1, wobei die Verbindung der Formel 1 oder 2 eine Verbindung von Formel 1-3 oder 2-3 ist: 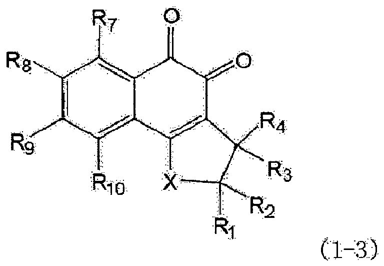 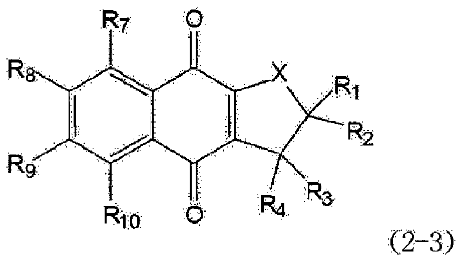 wobei R₁ bis R₁₀ und X dieselben Bedeutungen aufweisen wie in Anspruch 1 definiert.

7. Zusammensetzung zur Verwendung nach Anspruch 1, wobei die Verbindung der Formel 1 oder 2 eine Verbindung der Formel 1-4 oder 2-4 ist: 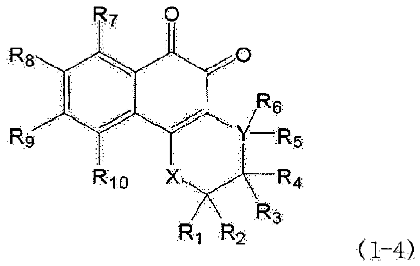 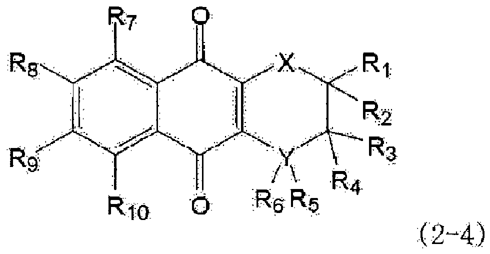 wobei R₁ bis R₁₀, X und Y dieselben Bedeutungen aufweisen wie in Anspruch 1 definiert.

8. Zusammensetzung zur Verwendung nach Anspruch 1, wobei die Verbindung der Formel 1 oder 2 eine Verbindung der Formel 1-5, 1-6, 2-5 oder 2-6 ist: 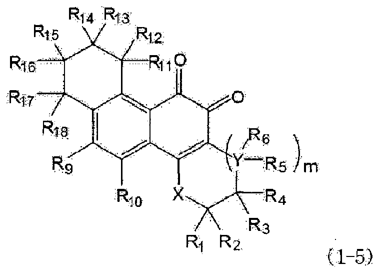 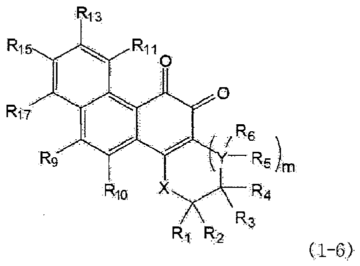 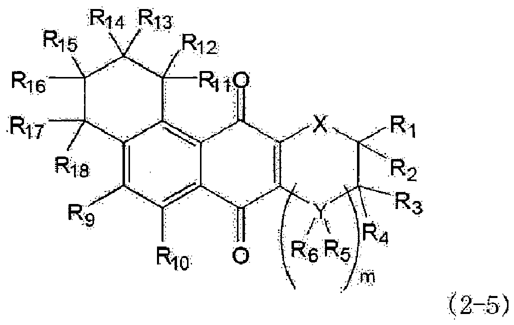 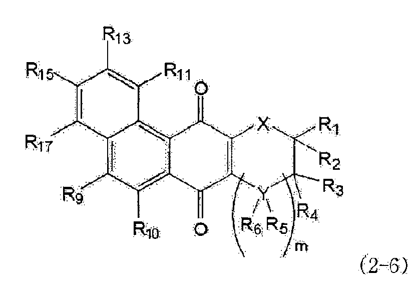 wobei
R₁ bis R₁₀, X, Y und m dieselben Bedeutungen wie in Anspruch 1 definiert haben, und R₁₁ bis R₁₈ jeweils unabhängig ausgewählt sind aus der Gruppe bestehend aus Wasserstoff, Hydroxy, Halogen, C₁-C₁₀ Alkyl, C₂-C₁₀ Alkenyl, C₂-C₁₀ Alkinyl, C₁-C₁₀ Alkoxy, C₁-C₁₀ Alkoxycarbonyl, C₁-C₁₀ Alkylamino, C₃-C₈ Cycloalkyl, C₃-C₈ Heterocycloalkyl, C₄-C₁₀ Aryl und C₄-C₁₀ Heteroaryl.

9. Zusammensetzung zur Verwendung nach Anspruch 8, wobei R₁₁ bis R₁₈ jeweils unabhängig ausgewählt sind aus der Gruppe bestehend aus Wasserstoff, Hydroxy, Halogen, C₁-C₁₀ Alkyl, C₂-C₁₀ Alkenyl, C₂-C₁₀ Alkinyl, C₁-C₁₀ Alkoxy, C₃-C₈ Cycloalkyl und Phenyl.

10. Zusammensetzung zur Verwendung nach Anspruch 7, wobei R1 und R₂ gleich C₁-C₁₀ Alkyl sind,
R₃ bis R₁₀ Wasserstoff sind,
X gleich O ist, und
Y gleich C ist.

11. Zusammensetzung zur Verwendung nach Anspruch 1, wobei der Hörverlust altersbedingte Presbyakusis ist.

12. Zusammensetzung zur Verwendung nach Anspruch 1, wobei der Hörverlust ototoxischer, arzneimittelbedingter Hörverlust ist.

13. Zusammensetzung zur Verwendung nach einem der Ansprüche 1 bis 12, die eine pharmazeutische Zusammensetzung ist, umfassend die Verbindung der Formel 1 oder 2 oder ein pharmazeutisch akzeptables Salz, Solvat oder Isomer davon, zusammen mit einem pharmazeutisch akzeptablen Träger.

14. Zusammensetzung zur Verwendung nach einem der Ansprüche 1 bis 12, die ein funktionelles Nahrungsmittel ist, umfassend die Verbindung der Formel 1 oder 2 oder ein pharmazeutisch akzeptables Salz, Solvat oder Isomer davon, zusammen mit einem sitologisch akzeptablen Träger.

15. Zusammensetzung zur Verwendung nach Anspruch 1, wobei die Verbindung eine Verbindung der Formel: 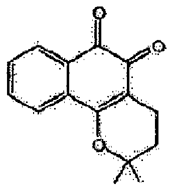 ist.

## Revendications

1. Composition comprenant un composé de Formule 1 ou 2, ou un sel, solvate ou isomère pharmaceutiquement acceptable de celui-ci pour une utilisation dans le traitement ou la prévention de la perte d'audition choisie parmi la presbyacousie liée à l'âge et la perte d'audition due à des médicaments ototoxiques : 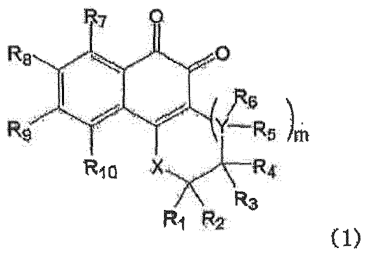 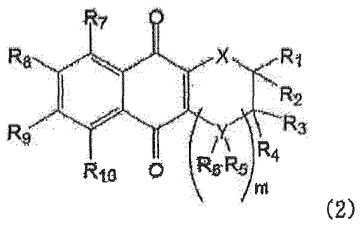 formules dans lesquelles
R₁ à R₆ sont chacun indépendamment choisis dans le groupe constitué de l'atome d'hydrogène, d'un groupe hydroxy, d'un atome d'halogène, et des groupes amino, alkylamino en C₁ à C₁₀, substitué ou non substitué, dialkylamino en C₁ à C₁₀, substitué ou non substitué, alkyle en C₁ à C₁₀, substitué ou non substitué, alcényle en C₂ à C₁₀, substitué ou non substitué, alcynyle en C₂ à C₁₀, substitué ou non substitué, alcoxy en C₁ à C₁₀, substitué ou non substitué, alcoxycarbonyle en C₁ à C₁₀, substitué ou non substitué, acyle en C₁ à C₁₀, substitué ou non substitué, cycloalkyle en C₃ à C₈, substitué ou non substitué, hétérocycloalkyle en C₃ à C₈, substitué ou non substitué, aryle en C₄ à C₁₀, substitué ou non substitué, hétéroaryle en C₄ à C₁₀, substitué ou non substitué, et -(CH₂)ₙ-aryle, substitué ou non substitué, ou deux substituants de R₁ à R₆ peuvent être pris ensemble pour former une double liaison ou une structure cyclique en C₃ à C₆, substituée ou non substituée, qui peut être saturée ou partiellement ou complètement insaturée ; le substituant étant au moins un substituant choisi dans le groupe constitué d'un groupe hydroxy, d'un atome d'halogène, et des groupes alkyle en C₁ à C₁₀, alcényle en C₂ à C₁₀, alcynyle en C₂ à C₁₀, alcoxy en C₁ à C₁₀, alcoxycarbonyle en C₁ à C₁₀, alkylamino en C₁ à C₁₀, cycloalkyle en C₃ à C₈, hétérocycloalkyle en C₃ à C₈, aryle en C₄ à C₁₀ et hétéroaryle en C₄ à C₁₀ ;
R₇ à R₁₀ sont chacun indépendamment choisis dans le groupe constitué de l'atome d'hydrogène, d'un groupe hydroxy, d'un atome d'halogène, et des groupes amino, alkylamino en C₁ à C₁₀, substitué ou non substitué, dialkylamino en C₁ à C₁₀, substitué ou non substitué, alkyle en C₁ à C₁₀, substitué ou non substitué, alcényle en C₂ à C₁₀, substitué ou non substitué, alcynyle en C₂ à C₁₀, substitué ou non substitué, alcoxy en C₁ à C₁₀, substitué ou non substitué, alcoxycarbonyle en C₁ à C₁₀, substitué ou non substitué, acyle en C₁ à C₁₀, substitué ou non substitué, cycloalkyle en C₃ à C₈, substitué ou non substitué, hétérocycloalkyle en C₃ à C₈, substitué ou non substitué, aryle en C₄ à C₁₀, substitué ou non substitué, hétéroaryle en C₄ à C₁₀, substitué ou non substitué, et -(CH₂)ₙ-aryle, substitué ou non substitué, ou deux substituants de R₇ à R₁₀ peuvent être pris ensemble pour former une structure cyclique en C₃ à C₆, substituée ou non substituée, qui peut être saturée ou partiellement ou complètement insaturée : le substituant étant au moins un substituant choisi dans le groupe constitué d'un groupe hydroxy, d'un atome d'halogène, et des groupes alkyle en C₁ à C₁₀, alcényle en C₂ à C₁₀, alcynyle en C₂ à C₁₀, alcoxy en C₁ à C₁₀, alcoxycarbonyle en C₁ à C₁₀, alkylamino en C₁ à C₁₀, cycloalkyle en C₃ à C₈, hétérocycloalkyle en C₃ à C₈, aryle en C₄ à C₁₀ et hétéroaryle en C₄ à C₁₀;
X représente O, S ou NR', avec R' représentant un atome d'hydrogène ou un groupe alkyle en C₁ à C₆ ;
Y représente C, S, N ou O, à condition que lorsque Y représente S ou O, R₅ et R₆ ne représentent pas un substituant, et lorsque Y représente N, R₅ représente un atome d'hydrogène ou un groupe alkyle en C₁ à C₆ et R₆ ne représente pas un substituant ;
m vaut 0 ou 1, à condition que lorsque m vaut 0, les atomes de carbone adjacents à m forment une structure cyclique par l'intermédiaire d'une liaison directe ; et
n représente un nombre entier de 0 à 10.

2. Composition pour une utilisation selon la revendication 1, dans laquelle X représente O ou S, et Y représente C ou O.

3. Composition pour une utilisation selon la revendication 1, dans laquelle R₁ à R₆ sont chacun indépendamment choisis dans le groupe constitué de l'atome d'hydrogène, d'un groupe hydroxy, d'un atome d'halogène, et des groupes alkyle en C₁ à C₁₀, substitué ou non substitué, alcényle en C₂ à C₁₀, substitué ou non substitué, alcoxy en C₁ à C₁₀, substitué ou non substitué et -(CH₂)ₙ-phényle, ou R₁ et R₄ ou R₂ et R₃ sont pris ensemble pour former une double liaison ou une structure cyclique en C₄ à C₆, le substituant étant un groupe alkyle en C₁ à C₁.

4. Composition pour une utilisation selon la revendication 1, dans laquelle R₇ à R₁₀ sont chacun indépendamment choisis dans le groupe constitué de l'atome d'hydrogène, d'un groupe hydroxy, d'un atome d'halogène, et des groupes alkyle en C₁ à C₁₀, substitué ou non substitué, et alcoxy en C₁ à C₁₀, substitué ou non substitué.

5. Composition pour une utilisation selon la revendication 1, dans laquelle le composé de Formule 1 ou 2 est un composé de Formule 1-1, 2-1, 1-2 ou 2-2 : 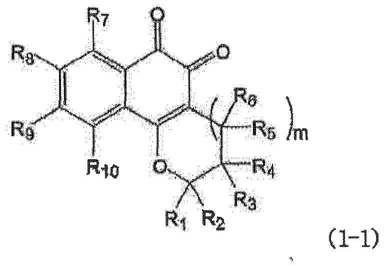 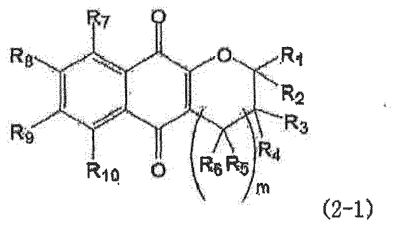 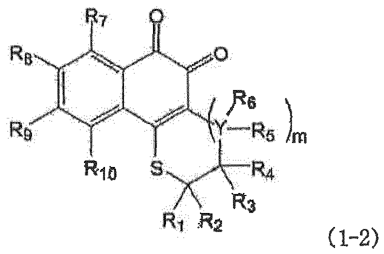 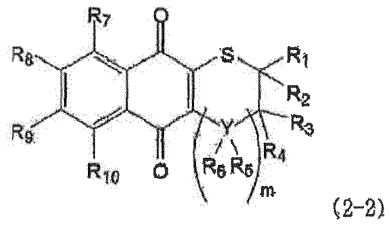 formules dans lesquelles R₁ à R₁₀, Y et m ont les mêmes significations que définies dans la revendication 1.

6. Composition pour une utilisation selon la revendication 1, dans laquelle le composé de Formule 1 ou 2 est un composé de Formule 1-3 ou 2-3 : 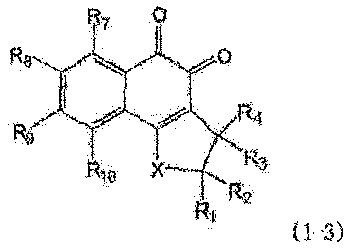 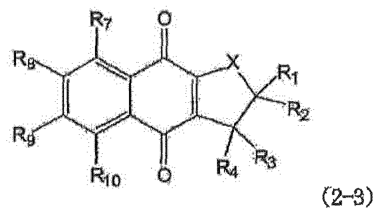 formules dans lesquelles R₁ à R₁₀, et X ont les mêmes significations que définies dans la revendication 1.

7. Composition pour une utilisation selon la revendication 1, dans laquelle le composé de Formule 1 ou 2 est un composé de Formule 1-4 ou 2-4 : 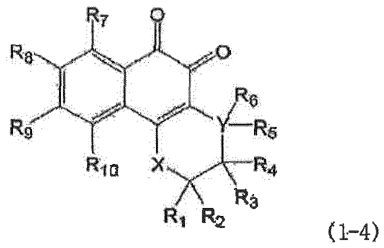 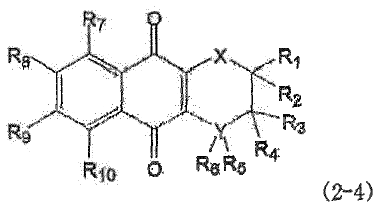 formules dans lesquelles R₁ à R₁₀, X et Y ont les mêmes significations que définies dans la revendication 1.

8. Composition pour une utilisation selon la revendication 1, dans laquelle le composé de Formule 1 ou 2 est un composé de Formule 1-5, 1-6, 2-5 ou 2-6 : 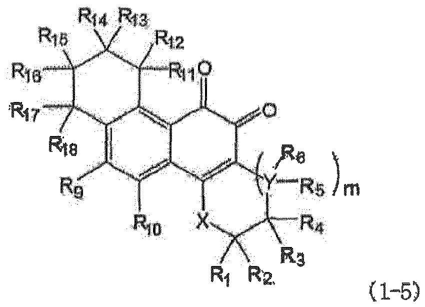 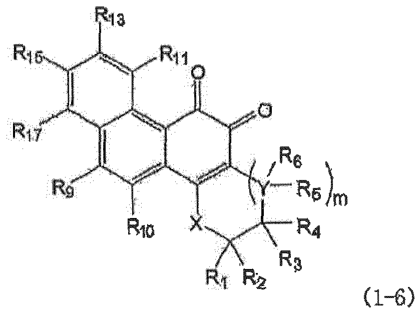 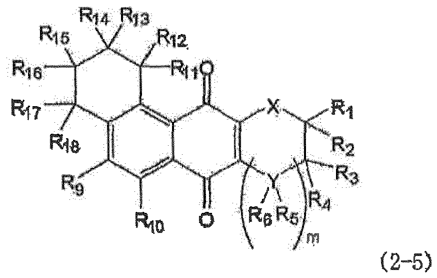 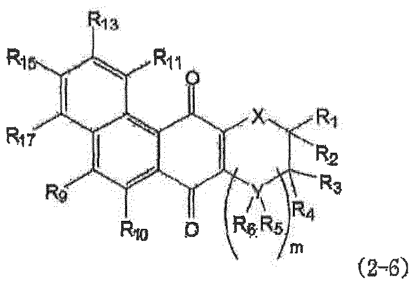 formules dans lesquelles
R₁ à R₁₀, X, Y et m ont les mêmes significations que définies dans la revendication 1, et
R₁₁ à R₁₈ sont chacun indépendamment choisis dans le groupe constitué de l'atome d'hydrogène, d'un groupe hydroxy, d'un atome d'halogène, et des groupes alkyle en C₁ à C₁₀, alcényle en C₂ à C₁₀, alcynyle en C₂ à C₁₀, alcoxy en C₁ à C₁₀, alcoxycarbonyle en C₁ à C₁₀, alkylamino en C₁ à C₁₀, cycloalkyle en C₃ à C₈, hétérocycloalkyle en C₃ à C₈, aryle en C₄ à C₁₀ et hétéroaryle en C₄ à C₁₀.

9. Composition pour une utilisation selon la revendication 8, dans laquelle R₁₁ à R₁₈ sont chacun indépendamment choisis dans le groupe constitué de l'atome d'hydrogène, d'un groupe hydroxy, d'un atome d'halogène, et des groupes alkyle en C₁ à C₁₀, alcényle en C₂ à C₁₀, alcynyle en C₂ à C₁₀, alcoxy en C₁ à C₁₀, cycloalkyle en C₃ à C₈ et phényle.

10. Composition pour une utilisation selon la revendication 7, dans laquelle
R₁ et R₂ représentent un groupe alkyle en C₁ à C₁₀,
R₃ à R₁₀ représentent un atome d'hydrogène,
X représente 0, et
Y représente C.

11. Composition pour une utilisation selon la revendication 1, dans laquelle la perte d'audition est la presbyacousie liée à l'âge.

12. Composition pour une utilisation selon la revendication 1, dans laquelle la perte d'audition est la perte d'audition due à des médicaments ototoxiques.

13. Composition pour une utilisation selon l'une quelconque des revendications 1 à 12, qui est une composition pharmaceutique comprenant le composé de Formule 1 ou 2, ou un sel, solvate ou isomère pharmaceutiquement acceptable de celui-ci conjointement avec un vecteur pharmaceutiquement acceptable.

14. Composition pour une utilisation selon l'une quelconque des revendications 1 à 12, qui est un aliment fonctionnel comprenant le composé de Formule 1 ou 2, ou un sel, solvate ou isomère pharmaceutiquement acceptable de celui-ci conjointement avec un vecteur sitologiquement acceptable.

15. Composition pour une utilisation selon la revendication 1, dans laquelle le composé est un composé de Formule :
